(19) Europäisches Patentamt
European Patent Office
Office européen des brevets

(11) Veröffentlichungsnummer : **0 137 478**
**B1**

(12) **EUROPÄISCHE PATENTSCHRIFT**

(45) Veröffentlichungstag der Patentschrift : **29.07.87**

(51) Int. Cl.⁴ : **C 07 D295/02**

(21) Anmeldenummer : **84112010.8**

(22) Anmeldetag : **06.10.84**

(54) **Verfahren zur Herstellung von N-methylierten cyclischen Iminen.**

(30) Priorität : **13.10.83 DE 3337182**

(43) Veröffentlichungstag der Anmeldung : **17.04.85 Patentblatt 85/16**

(45) Bekanntmachung des Hinweises auf die Patenterteilung : **29.07.87 Patentblatt 87/31**

(84) Benannte Vertragsstaaten : **BE DE FR GB IT NL**

(56) Entgegenhaltungen :
**DE-A- 2 813 162**
**GB-A- 1 106 084**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Hertel, Otto, Dr.**
**Koenigsbacher Strasse 68**
**D-6700 Ludwigshafen (DE)**
Erfinder : **Schroeder, Wolfgang, Dr.**
**Seebacher Strasse 51**
**D-6702 Bad Duerkheim (DE)**
Erfinder : **Voges, Dieter, Dr.**
**Richard-Wagner-Strasse 28**
**D-6800 Mannheim 1 (DE)**

EP 0 137 478 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von N-methylierten cyclischen Iminen durch Umsetzung von Diolen mit Monomethylamin an einem Kupferkatalysator.

Üblicherweise werden N-methylierte cyclische Imine aus Diolen dadurch hergestellt, daß man zunächst durch Umsetzung mit Ammoniak das freie Imin erzeugt und anschließend, nach dessen Reinigung, die Methylierung mit Formaldehyd oder Methanol unter hydrierenden Bedingungen vornimmt, vgl. US-A-3 167 551 und BE-A-694 068. Diese Verfahren verlaufen aber über mehrere Stufen und erfordern neben hohem technischem Aufwand zusätzlich auch einen großen Energieaufwand.

Nach dem Vorschlag der US-A-3 151 113 setzt man das jeweilige Diol mit Ammoniak und Methanol unter gleichzeitiger Anwesenheit von Wasserstoff an einem Hydrierkatalysator um. Dabei wird ein Katalysator verwendet, dessen aktive Komponente überwiegend aus Nickel besteht. Als Reaktionsprodukt erhält man hier ein Gemisch aus N-Methylmorpholin, Morpholin, nicht umgesetzten Ausgangsprodukten sowie Nebenprodukten.

Gemäß der BE-A-842 461 wird das Diol mit Methylamin in flüssiger Phase in Gegenwart einer Phosphorverbindung zum Zielprodukt umgesetzt. Hierbei benötigt man aber hohe Temperaturen (220 bis 350 °C) und auch einen hohen Druck (bis zu 280 bar).

Auch bei weiteren Verfahren, bei denen Diole mit Alkylaminen umgesetzt werden, wird hoher Druck benötigt.

In der US-A-3 709 881 wird dabei z. B. die Verwendung eines Nickelkatalysators vorgeschlagen, wobei aber eine Ausbeute an N-Alkylmorpholin von lediglich 42,5 % erreicht wird.

In einem anderen Fall (GB-A-1 106 084) enthält der Katalysator Kupfer, Nickel, Kobalt oder Chrom. Die Umsetzung von Diethylenglykol mit Methylamin wird bei hohem Druck durchgeführt (30 bis 400 bar, insbesondere 100 bis 200 bar).

In diesen bekannten Verfahren findet die Umsetzung in flüssiger Phase statt. Trotz sehr langer Reaktionszeiten (bis 6,5 Stunden) sind die erzielten Ausbeuten dabei wenig befriedigend.

Aufgabe der vorliegenden Erfindung war es nun, ein Verfahren zur Herstellung von N-methylierten cyclischen Iminen durch Aminierung eines zur Ringbildung befähigten Diols mit Monomethylamin in Gegenwart eines kupferhaltigen Katalysators und in Gegenwart von Wasserstoff bei erhöhter Temperatur und unter Druck bereitzustellen, das die geschilderten Nachteile vermeidet und die Zielprodukte in guten Ausbeuten liefert.

Diese Aufgabe wird gelöst bei einem Verfahren der genannten Art, das dadurch gekennzeichnet ist, daß man die Umsetzung in der Gasphase bei einem Druck von 1 bis 50 bar und einer Temperatur von 150 bis 250 °C durchführt und einen Katalysator verwendet, wie er durch Temperung eines basischen Kupfer und Aluminium enthaltende Carbonats der allgemeinen Zusammensetzung

$$Cu_mAl_6(CO_3)_{0,5m}O_3(OH)_{m+12},$$

worin in einem beliebigen, auch nicht ganzzahligen Wert zwischen 2 und 6 bedeutet, bei einer Temperatur von 350 bis 700 °C erhältlich ist.

Dabei kommen solche Diole in Betracht, die in der Lage sind, mit Monomethylamin eine Ringschlußreaktion einzugehen beispielsweise Alkandiole mit geradkettiger oder verzweigter Kohlenstoffkette wie Butan-1, 4-diol, Pentan-1, 5-diol, Hexan-1, 6-diol oder auch Alkandiole deren Kohlenstoffkette durch Heteroatome unterbrochen ist z. B. Ethylendiglykol oder Diethanolamin.

Das erfindungsgemäße Verfahren setzt sich aus folgenden Schritten zusammen :

Zuführung der Rohstoffe (Diol, Monomethylamin, Wasserstoff)

Verdampfung der Rohstoffe (Diol)

katalytische Umsetzung der Rohstoffe, möglichst mit Abführung der Reaktionswärme

Verflüssigung der kondensierbaren Reaktionsprodukte

Rückführung der nicht kondensierbaren Anteile (Wasserstoff) zum Verdampfer (diese werden im folgenden als Kreisgas bezeichnet)

Abtrennung des N-methylierten cyclischen Imins vom Kondensat und seine Reinigung.

Für die Zuführung der Rohstoffe werden die in der Technik üblichen Geräte verwendet. Dabei werden je Mol Diol 2 bis 40 Mol, insbesondere 5 bis 30 Mol, Monomethylamin eingesetzt, die mit dem Diol vermischt werden. Zur Erhaltung der Gasphase beim Durchströmen des Katalysatorbettes wird ein im wesentlichen aus Wasserstoff bestehender Gasstrom verwendet, dessen Menge zwischen 200 und 1.000 Druck-Liter (Nl pro Absolutdruck (bar)) je Mol Diol beträgt.

Die Verdampfung des Diols führt man vorteilhaft so durch, daß man das überwiegend aus Wasserstoff bestehende Kreisgas vorwärmt und zusammen mit dem ebenfalls vorgewärmten Diol und dem Monomethylamin in den Verdampfer einleitet. Dabei wird die zur Verdampfung notwendige Wärme dem Verdampfer indirekt in Form von Wasserdampf oder umlaufendem heißen Öl (das in einer externen Wärmequelle erhitzt wird) oder durch die Verbrennung z. B. von Gas oder Öl zugeführt. Das aus dem Verdampfer tretende Dampfgemisch bestehend aus Wasserstoff, Diol und Monomethylamin besitzt eine Temperatur zwischen 150 und 250 °C.

Die katalytische Umsetzung der Rohstoffe geschieht in einem Reaktor, der für die Zu- und Abfuhr von Wärme eingerichtet ist. Bevorzugt wird dabei ein Röhrenreaktor, der beispielsweise den Katalysator innerhalb der Rohre enthält, während der Raum außerhalb der Rohre für die

Zu- und Abfuhr von Wärme dient. Anstelle eines solchen Festbettreaktors kann auch ein Wirbelschichtreaktor verwendet werden. Um den Röhrenreaktor auf die vorgesehene Reaktionstemperatur einzustellen, kann der Raum außerhalb der Rohre beispielsweise einen Anschuß zum Einblasen von Wasserdampf besitzen oder mit Wasser zur Siedekühlung gefüllt sein. Die Reaktionstemperatur liegt im allgemeinen zwischen 150 und 250 °C, vorzugsweise zwischen 180 und 220 °C. Der genaue Wert der Temperatur wird bestimmt von der Natur des verwendeten Diols und von der spezifischen Belastung des Reaktors. Dabei gilt die Regel, daß die Temperatur umso niedriger sein kann, je höher der Dampfdruck des Diols ist und je niedriger die spezifische Belastung des Reaktors ist.

Der im Reaktor einzuhaltende Druck liegt im allgemeinen zwischen 1 und 50 bar, bevorzugt wird ein Druck zwischen 5 und 25 bar und insbesondere zwischen 5 und 20 bar. Der genaue Wert des Drucks richtet sich nach den individuellen Eigenschaften der Diole, eine Regel läßt sich hier nicht aufstellen.

Man verwendet einen Katalysator, der durch Temperung eines basischen Kupfer und Aluminium enthaltenden Carbonats der allgemeinen Zusammensetzung

$$Cu_mAl_6(CO_3)_{0,5m}O_3(OH)_{m+12},$$

wobei m einen beliebigen, auch nicht ganzzahligen Wert zwischen 2 und 6 bedeutet, bei einer Temperatur von 350 bis 700 °C erhältlich ist. Die Herstellung von solchen basischen Kupfer und Aluminium enthaltenden Carbonaten ist in der DE-B-24 45 303 beschrieben.

Die getemperte Verbindung kann dann durch Mahlen und Aussieben auf eine geeignete Korngrößenverteilung in eine katalytisch besonders aktive Form übergeführt werden. Sie läßt sich aber auch durch bekannte Methoden in die Form von Strängen, Pillen, Kugeln, Ringen, etc. bringen. Ihre Aktivierung erfolgt durch Reduktion, wobei man die Reduktion zweckmäßig in einer der eigentlichen Umsetzung vorgelagerten Stufe vornimmt. Als Reduktionsgas verwendet man zweckmäßig ein Wasserstoff/Stickstoff-Gemisch, das 5 bis 10 Vol.% Wasserstoff enthält. Die Reduktionstemperatur beträgt 140 bis 200 °C.

Das in den Reaktor strömende Gas weist im allgemeinen folgende Zusammensetzung auf : 80 bis 98 Mol.% Wasserstoff, 1,5 bis 19 Mol.% Monomethylamin und 0,2 bis 1 Mol.%Diol. Seine Strömungsmenge ist so bemessen, daß die mittlere Verweilzeit (berechnet aus der nach Druck und Temperatur korrigierten Gasmenge und bezogen auf den leer gedachten Reaktor) zwischen 1 und 30 Sekunden, insbesondere zwischen 2 und 15 Sekunden beträgt. In dieser Zeit wird das Diol im allgemeinen vollständig ind as N-methylierte cyclische Imin übergeführt. Außer dem weiteren Reaktionsprodukt Wasser entstehen geringe, bilanzmäßig oft unbedeutende Mengen an Nebenprodukten.

Die Abtrennung der Reaktionsprodukte vom Kreisgas erfolgt durch Kühlung, Kondensation und Abscheidung des Kondensats. Vorzugsweise führt man die Kühlung in zwei Temperaturbereichen durch. Der erste Bereich, der im allgemeinen bei 50 bis 150 °C liegt, ist so gewählt, daß die Hauptmenge der im Gas enthaltenen kondensierbaren Stoffe verflüssigt wird (zwischen 50 und 90 %).

Im zweiten Bereich wird die Temperatur so weit gesenkt, daß auch die Restmenge der noch im Gas enthaltenen kondensierbaren Stoffe verflüssigt wird. Ein kleiner Teil bleibt entsprechend seinem Partialdruck in der Gasphase zurück. Der zweite Temperaturbereich liegt im allgemeinen zwischen 0 und 50 °C. So wird erreicht, daß Monomethylamin, das im Kreisgas in stöchiometrischem Überschuß vorliegt, nur zu einem kleinen Teil im Kondensat gelöst wird. Die vereinigten Kondensate enthalten im allgemeinen weniger als 10 Gew.% Monomethylamin.

Neben der Kondensation in zwei verschiedenen Temperaturbereichen ist auch eine einstufige Kondensation möglich. Sie hat aber den Nachteil, daß die im Kondensat gelöste Menge an Monomethylamin wesentlich größer ist und im allgemein in einem Bereich um 20 Gew.% liegt.

Von dem nach Abtrennung der Reaktionsprodukte erhaltenen Kreisgas wird ein kleiner Teil als Abgas dem Kreislauf entzogen, um die Anreicherung unerwünschter Verunreinigungen zu vermeiden. Diese Abgasmenge beträgt im allgemeinen zwischen 0,1 und 5 % der Kreisgasmenge.

Die Hauptmenge des Kreisgases wird, entsprechend den Umsetzungsbedingungen, wieder mit Wasserstoff und Monomethylamin vermischt und die resultierende Mischung erneut dem Verdampfer zugeführt.

Der Umsatz des Diols ist normalerweise vollständig. Seine Abtrennung von den Reaktionsprodukten sowie seine Reinigung und Rückführung zum Reaktor erübringen sich also.

Die gewünschten Reaktionsprodukte, N-methylierte cyclische Imine, bilden mit Wasser im allgemeinen tiefsiedende Azeotrope. Diese Eigenschaft läßt sich zur Abtrennung von Nebenprodukten mit Vorteil ausnützen.

Die so erreichten Ausbeuten sind stets größer als 80 % der Theorie und erreichen meistens Werte über 90 %.

N-methylierte cyclische Imine sind wichtige Zwischenprodukte beispielsweise für die Synthese von Parmazeutika, Pflanzenschutzmittel oder Farbstoffen.

Die folgenden Beispiele sollen die Erfindung erläutern.

Beispiel 1

Synthese von N-Methylmorpholin

Man verwendete eine Apparatur mit folgenden Bestandteilen : a) einem Einrohrreaktor mit 2 l Katalysator (hergestellt gemäß DE-AS 24 45 303 - und bei 140 bis 200 °C mit eine $H_2/N_2$-gemisch (5

bis 10 Vol.% $H_2$) reduziert) und darüber 1 l Füllkörper, b) Kühler, c) Abscheider, d) Kreisgaspumpe, e) Wärmetauschern zum Heizen des Kreisgases und der sonstigen Zuläufe zum Reaktor, f) Pumpe für die flüssigen Zuläufe, g) Verdampfer für die flüssigen Zuläufe, h) Meßeinrichtungen für die Zuläufe für das Kreisgas und das Abgas sowie i) Thermostaten für die unter a), b), e) und g) genannten Apparate.

Die mit den Produktströmen in Berührung kommenden Teile bestanden aus Edelstahl und waren für einen erhöhten Arbeitsdruck ausgelegt. Die Wärmetauscher, der Verdampfer und die Kühler bestanden aus ummantelten Rohren, der Verdampfer enthielt Einbauten zur Vergrößerung der Fläche. Durch das Innenrohr strömte das Reaktionsmedium, durch den Mantel das Wärmeträgeröl.

Im laufenden Betrieb wurde das zu den Vorheizern geführte Wärmeträgeröl bei einer Temperatur von 200 °C gehalten, das zum Verdampfer geführte Wärmeträgeröl besaß eine Temperatur von 220 °C, so daß das Gasgemisch mit einer Temperatur von etwa 200 °C zum Reaktor strömte. Das in den Verdampfer geführte Gasgemisch bestand zu 95 Vol.% aus Wasserstoff und zu 5 Vol.% aus Monomethylamin. Die stündlich durchgesetzte Menge betrug 10 $Nm^3$, der Druck wurde bei 10 bar gehalten. Vor dem Verdampfer wurde dem Gasstrom vorgeheiztes Diethylenglykol in einer Menge von 0,2 l pro Stunde zugemischt.

Die Temperatur des von oben in den Reaktor eintretenden Gasgemisches stellte sich in der Füllkörperschicht auf die Reaktionstemperatur von 200 °C ein. In der Katalysatorschicht fand dann die Reaktion von Diethylenglykol mit Monomethylamin statt. Die Reaktionsprodukte wurden kondensiert, vom nicht kondensierbaren Gas getrennt und abgeschieden. Vom Kreisgas wurden 100 Nl/h (= 1 % der Gesamtmenge) als Abgas abgeführt, der Rest wurde mit der Kreisgaspumpe zum Kreisgasvorheizer zurückgefördert. Die durch die Reaktion, durch Lösung im Kondensat und die im Abgas aus dem Kreislauf abgeführten Stoffmengen wurden ergänzt und damit der Kreislauf geschlossen.

Das Kondensat wurde sowohl durch fraktionierende Destillation als auch gaschromatographisch untersucht. Man fand folgende Zusammensetzung : N-Methylmorpholin 56 Gew.%, Wasser 22 Gew.%, Nebenprodukte 5 Gew.%, gelöstes Monomethylamin 17 Gew.%. Dies ergibt eine Ausbeute von etwa 92 % der Theorie.

Beispiel 2

Synthese von N-Methylpiperidin

Die im beispiel 1 beschriebene Apparatur wurde wie folgt betrieben : Die temperatur im Reaktor wurde bei 180 °C und der Druck bei 5 bar gehalten. Das Gasgemisch zum Verdampfer bestand zu 95 Vol.% aus Wasserstoff und zu 5 Vol.% aus Monomethylamin, die stündlich durchgesetzte Menge betrug 5 $Nm^3$. Vor dem Verdampfer wurde dem Gasstrom vorgeheiztes technisches Pentan-1,5-Diol (Reinheit ca. 96 Gew.%) in einer Menge von 0,2 l pro Stunde zugemischt.

Das Reaktionsprodukt wurde kondensiert, abgeschieden und sowohl durch fraktionierende Destillation als auch gaschromatographisch untersucht.

Man fand folgende Zusammensetzung : N-Methylpiperidin 56 Gew.%, Wasser 22 Gew.%, Nebenprodukte 4 Gew.%, Monomethylamin 18 Gew.%. Dies ergibt eine Ausbeute von etwa 95 % der Theorie.

**Patentansprüche**

1. Verfahren zur Herstellung von N-methylierten cyclischen Iminen durch Aminierung eines zur Ringschlußreaktion befähigten Diols mit Monomethylamin in Gegenwart eines kupferhaltigen Katalysators und in Gegenwart von Wasserstoff bei erhöhter Temperatur und unter Druck, dadurch gekenzeichnet, daß man die Umsetzung in der Gasphase bei einem Druck von 1 bis 50 bar und einer Temperatur von 150 bis 250 °C durchführt und einen Katalysator verwendet, wie er durch Temperung eines basischen Kupfer und Aluminium enthaltende Carbonats der allgemeinen Zusammensetzung

$$Cu_mAl_6(CO_3)_{0.5\ m}O_3(OH)_{m\ +\ 12'}$$

worin in einem beliebigen, auch nicht ganzzahligen Wert zwischen 2 und 6 bedeutet, bei einer Temperatur von 350 bis 700 °C erhältlich ist.

2. Verfahren gemäß Anspruch 1, dadurch gekennzeichnet, daß man die Umsetzung bei einem Druck von 5 bis 25 bar durchführt.

3. Verfahren gemäß Anspruch 1, dadurch gekennzeichnet, daß man die Umsetzung bei einem Druck von 5 bis 20 bar durchführt.

4. Verfahren gemäß Anspruch 1, dadurch gekennzeichnet, daß man die Umsetzung bei einer Temperatur von 180 bis 220 °C durchführt.

**Claims**

1. A process for the preparation of an N-methylated cyclic imine by amination of a diol, capable of entering into a cyclization reaction, with monomethylamine in the presence of a copper-containing catalyst and in the presence of hydrogen at elevated temperature and under pressure, wherein the reaction is carried out in the gas phase under a pressure of from 1 to 50 bars and at a temperature of from 150 to 250 °C, and the catalyst used is one that is obtainable by heating a carbonate containing basic copper and aluminium and having the general composition

$$Cu_mAl_6(CO_3)_{0.5\ m}O_3(OH)_{m\ +\ 12'}$$

where m is any number of integer between 2 and

6, at a temperature of from 350 to 700 °C.

2. A process as claimed in claim 1, wherein the reaction is carried out at a pressure of from 5 to 25 bars.

3. A process as claimed in claim 1, wherein the reaction is carried out at a pressure of from 5 to 20 bars.

4. A process as claimed in claim 1, wherein the reaction is carried out at a temperature of from 180 to 220 °C.

**Revendications**

1. Procédé de préparation d'imines cycliques N-méthylées par l'amination d'un diol pouvant donner lieu à une cyclisation par la monométhylamine, à température accrue et sous pression en présence d'hydrogène et d'un catalyseur au cuivre, caractérisé en ce que la réaction est réalisée en phase gazeuse à une température comprise entre 150 et 250 °C et sous une pression de 1 à 50 bars, le catalyseur étant le produit obtenu par un traitement thermique à une température comprise entre 350 et 700 °C d'un carbonate basique, contenant du cuivre et de l'aluminium, de la composition générale

$$Cu_mAl_6(CO_3)_{0,5m}O_3(OH)_{m+12},$$

dans laquelle m possède une valeur quelconque, même décimale, entre 2 et 6.

2. Procédé suivant la revendication 1, caractérisé en ce que la réaction est réalisée sous une pression de 5 à 25 bars.

3. Procédé suivant la revendication 1, caractérisé en ce que la réaction est réalisée sous une pression de 5 à 20 bars.

4. Procédé suivant la revendication 1, caractérisé en ce que la réaction est réalisée à une température comprise entre 180 et 220 °C.